# EUROPEAN PATENT APPLICATION

(11) **EP 3 669 868 A1**
(43) Date of publication of application: **24.06.2020**
(21) Application number: 19215070.4
(22) Date of filing: 11.12.2019
(51) Int. Cl.: A61K 9/51, A61K 38/00

(54) **MALTODEXTRIN PARTICLES**

(30) Priority: 13.12.2018 LU 101054
(71) Applicant: Universität des Saarlandes, 66123 Saarbrücken (DE); PharmBioTec GmbH, 66123 Saarbruecken (DE)
(72) Inventor: Barthold, Sarah, 79541 Lörrach (DE); Groß, Henrik, 66128 Saarbruecken (DE); Hittinger, Marius, 66440 Blieskastel (DE); Primaveßy, Daniel, 66111 Saarbruecken (DE); Schneider, Marc, 66125 Saarbruecken (DE)
(74) Representative: Schiweck Weinzierl Koch Patentanwälte Partnerschaft mbB

(57) **Abstract**

The present invention relates to a method for the preparation of particles comprising the steps a) providing a solution A, which comprises water and Maltodextrin of the types DE 13.0 - 17.0 or DE 16.5 - 19.5, b) providing a solution B, which comprises acetone as and at least one poloxamer and c) combining solution A with solution B. Furthermore, the invention is directed to a particle comprising Maltodextrin and no protein, obtainable by said method, as well as to a particle comprising Maltodextrin and a protein, obtainable by said method if solution A further comprises at least one protein.

## Description

### TECHNICAL FIELD OF THE INVENTION

The present invention relates to a method for the preparation of particles comprising the steps a) providing a solution A, which comprises water and Maltodextrin of the types DE 13.0 - 17.0 or DE 16.5 - 19.5, b) providing a solution B, which comprises acetone and at least one poloxamer and c) combining solution A with solution B. Furthermore, the invention is directed to a particle comprising Maltodextrin and no protein, obtainable by said method, as well as to a particle comprising Maltodextrin and a protein, obtainable by said method if solution A further comprises at least one protein.

### BACKGROUND ART

Proteins and peptides are representing a growing class of active pharmaceutical ingredients (APIs). In order to overcome their unfavourable pharmacokinetic profiles, they are often applied as parenteral solution or encapsulated into drug delivery systems (DDS). PLGA and its derivatives are pharmaceutically known and approved polymers that have been utilized for the preparation of such DDS. However, the encapsulation of these highly hydrophilic proteins into the hydrophobic matrix of PLGA is often inefficient.

Polysaccharides instead are natural biopolymers having abundant resources in nature and usually low manufacturing costs. Additionally, they are mostly safe, non-toxic, hydrophilic and biodegradable, making them ideal candidates as material for the preparation of DDS. Starch, *e.g.* is one of the most studied polysaccharides for drug delivery. It consists of a complex macromolecular structure based on glucose units, forming amylose and amylopectin substructures. Maltodextrin is produced from starch by partial hydrolysis and is water soluble, in comparison to starch itself. It is a known excipient for different pharmaceutical applications, showing its biocompatibility. Thus, Maltodextrin is a potential carrier for hydrophilic active pharmaceutical ingredients, such as proteins and peptides. Different Maltodextrins are classified by dextrose equivalents (DE): A small DE indicates longer glucose chains while a high DE refers to smaller chains.

First attempts to form small particles were already successfully established, for example using spray drying, and self-assembly techniques with chitosan or gum Arabic. However, the preparation resulted either in large particles in the µm size range (1) or the preparation process involved toxic substances (2). Nanoprecipitation is an alternative method for small particle preparation and is known as a fast and mild technique, suitable for the encapsulation of proteins. Nanoprecipitation was first described by Fessi *et al*.(3) and later optimized for waxy maize starch acetate (SA) by Tan *et al.*(4)*.* In Tan *et al.* SA has been solved in acetone and a nano-suspension has been obtained by adding water dropwise.

Qui *et al.* (5) disclose the preparation of active polysaccharide-loaded maltodextrin nanoparticles by nanoprecipitation using absolute ethanol as anti-solvent. Ethanol is added dropwise to a solution of maltodextrin (dextrose equivalent, DE 10) and 0.5% (w/w) of an emulsifier (SDS, Tween 80 or Span 80) in water. Moreover, tea, pumpkin, and balsam pear polysaccharides were loaded on the maltodextrin nanoparticles.

Dimier-Poisson *et al.* (6), Paillard *et al* (7) and Bernocchi *et al.* (8) report the preparation of porous nanoparticles as delivery system, based on a cationic polysaccharide gel, obtained by derivatization of maltodextrin with epichlorhydrin and glycidyl-trimethyl-ammonium chloride (GTMA). Nanoparticles are obtained by crushing a gel intermediate with a high pressure homogenizer. The resulting cationic nanoparticles were mixed with 1,2-dipalmitoyl-sn-glycero-3-phosphatidylglycerol, loaded with bovine serum albumin (BSA) and tested as drug delivery systems.

In particular because of their potential use as drug delivery systems there is demand for methods for the preparation of suitable particles.

Thus, the aim of the present invention is to provide further particles and methods for their preparation.

### SUMMARY OF THE INVENTION

The invention is directed to a method for the preparation of particles, comprising the steps:
a) providing a solution A, which comprises water and maltodextrin of the types DE 13.0 - 17.0 or DE 16.5 -19.5;
b) providing a solution B, which comprises acetone and at least one poloxamer;
c) combining solution A and solution B.

Further, the invention comprises a particle comprising maltodextrin and no protein obtainable by said method. Furthermore, the invention is directed to a particle comprising maltodextrin and at least one protein, obtainable by said method wherein the solution in step a) further comprises at least one protein and its use in a drug delivery system.

The inventive method is mild, since particles in a nanometer size are formed at ambient pressure, without the need for high pressure homogenizers or high shear mechanical input. The method allows the preparation of particles comprising proteins or no proteins without further steps, depending only from the presensence or absence of the protein. These features render the inventive method particular suitable for scale-up. The inventive method allows the preparation of particles comprising no proteins in some embodiments in a intensity-weighted mean diameter (z-average) size of 80 to 100 nm and particles comprising proteins in some embodiments in a intensity-weighted mean diameter (z-average) size of 170 to 450 nm in a narrow size distribution and high encapsulation efficiencies. Moreover, the inventive particle formulations have been tested in respect to cytotoxicity and did not show any cytotoxic effects on A549 cells.

### BRIEF DESCRIPTION OF THE FIGURES

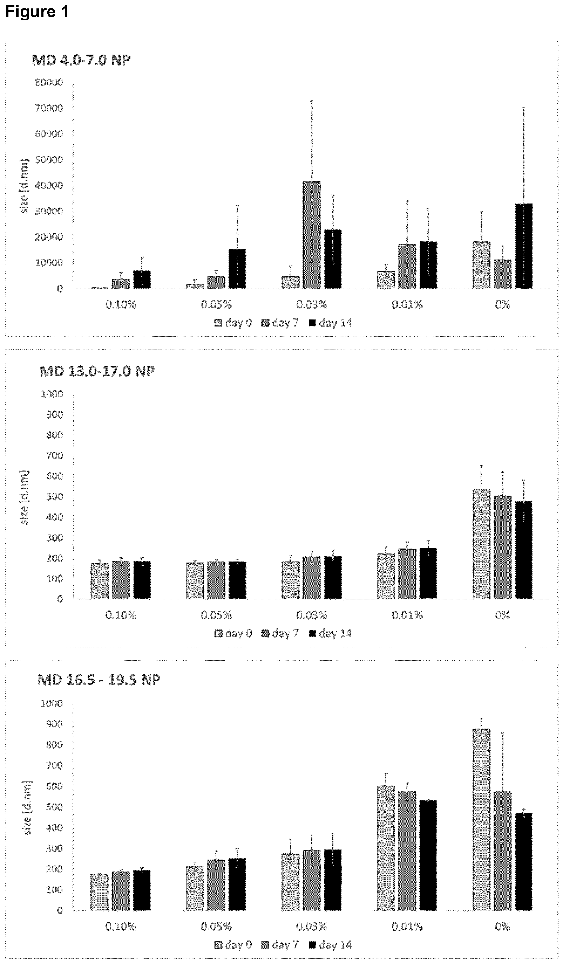

**Fig. 1****:** depicts a stability study of the prepared maltodextrin particles, using different amounts of stabilizer. Size was determined with the Zetasizer Nano ZS on the day of preparation (day 0), day 7 and day 14. Data are presented as mean ± SD of 3 batches.

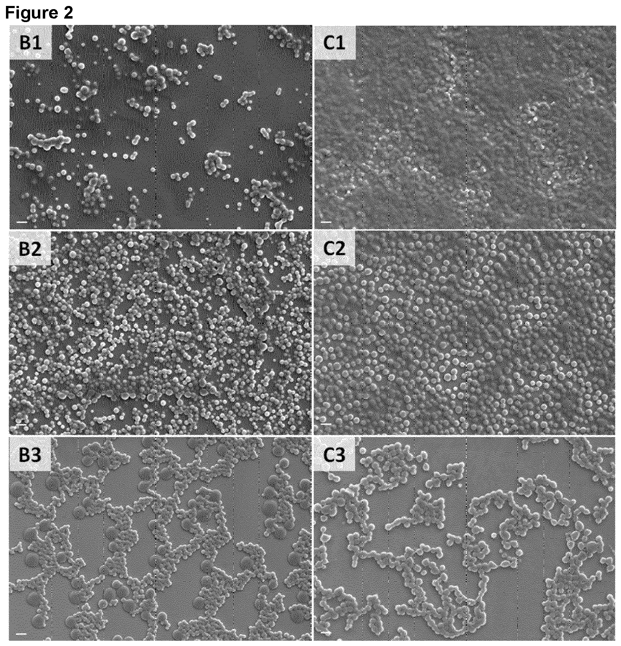

**Fig. 2** depicts SEM images of maltodextrin 13.0 -17.0 (B) - and maltodextrin 16.5 - 19.5 (C) particles, stabilized with poloxamer 0.1% (1), 0.05% (2), and 0.025% (3); scale bar = 1 µm. Particles are homogenously distributed but tend to fuse as a consequence of the reduced poloxamer concentration (B3).

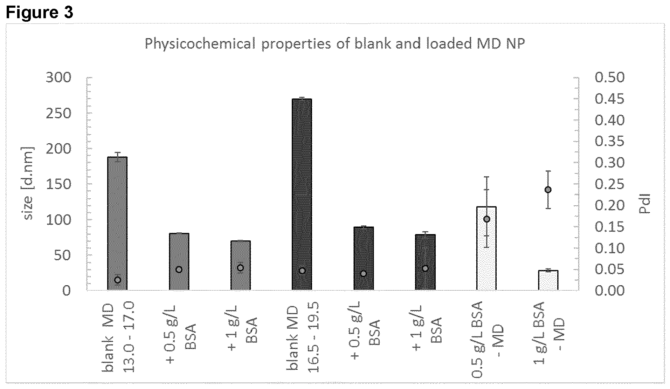

**Fig. 3** depicts physicochemical properties of blank and loaded maltodextrin 13.0 -17.0 (mid grey columns) and maltodextrin 16.5 - 19.5 (dark grey columns) particles. Loaded particles were prepared by coprecipitation with either 0.5 g/L BSA or 1 g/L BSA into a 0.025% poloxamer in acetone solution. Particles were homogenously distributed (Pdl < 0.05, represented by the orange dots), but showed smaller particle sizes of approximately 80 nm, when loaded with BSA. The precipitation of BSA without MD (light grey columns) showed high Pdl values (orange dots), indicating an inhomogeneous system.

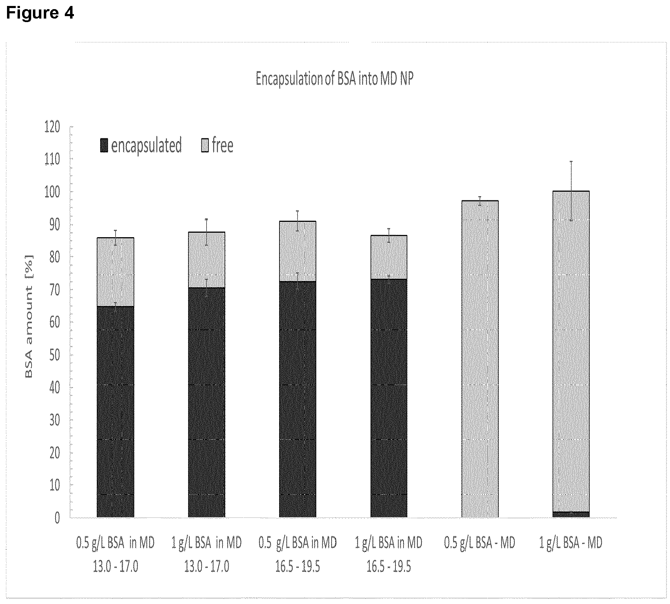

**Fig. 4** depicts the encapsulated amount of 0.5 g/L and 1 g/L BSA into maltodextrin 13.0 -17.0 and maltodextrin 16.5 - 19.5 particles. As control, BSA was also precipitated without maltodextrin (two columns on the right). All particles were prepared using a 0.025% poloxamer in acetone solution. All BSA could be found in the supernatant for the samples, precipitated without maltodextrin. For the samples, coprecipitated with maltodextrin a high amount of BSA (-70% of the applied BSA amount) could be found in the pellet.

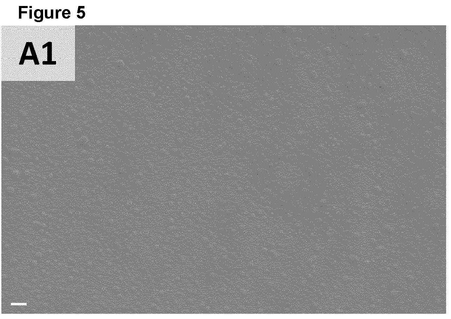

**Fig. 5** depicts the SEM image of maltodextrin 4.0 -7.0 (A) particles, stabilized with poloxamer 0.1% (1); scale bar = 1 µm.

### DETAILED DESCRIPTION OF THE INVENTION

The solution of the present invention is described in the following, exemplified in the appended examples, illustrated in the Figures and reflected in the claims.

The present invention relates to a method for the preparation of particles comprising the steps a), b) and c).

In step a), a solution A is provided which comprises water and maltodextrin of the types DE 13.0 - 17.0 or DE 16.5 - 19.5.

In the context of this invention, the term "providing of solution A" or "providing of solution B" means making available in any practical useful way for use in step c). This includes the actual preparation by combination of the ingredients and solvents, as well as the use of a potentially commercially or otherwise readily available solution according to the definitions of step a) and b).

Preferably, solution A is obtained by solving maltodextrin (CAS (Chemical Abstracts Service) Registry Number: 9050-36-6) of the types DE 13.0 - 17.0 or DE 16.5 - 19.5 in water.

Maltodextrin is a water soluble carbohydrate mixture prepared by hydrolysis of starch (poly-α-glucose). Maltodextrin is a mixture of monomers, dimers, oligomers and polymers of glucose. The respective composition is dependent from the degree of hydrolysis. The resulting hydrolysis products are characterized by dextrose equivalents. The dextrose equivalent (DE) of a polysaccharide mixture defines the weight percentage of reducing sugars, calculated as glucose, of the dry substance. Thus, it is equal to the mass of glucose, which has per 100 g dry substance the same reduction capacity. The "DE" - value of a starch hydrolysis product may be determined according to DIN ISO 5377 (1994-10) "Bestimmung des Reduktionsvermögens und des Dextroseäquivalentes von Stärkehydrolysaten". The DE of starch is 0, while that of glucose is 100. Maltrodextrin has a DE of 3 to 20.

Preferably, the concentration of Maltodextrin in solution A is 0.5 mg/ ml to 15 mg /ml, more preferably 1 mg/ml to 10 mg /ml, most preferably 5 mg/ml.

In solution A, water is a solvent for maltodextrin. Preferably, solution A comprises no other solvents than water.

In step a), the solution A optionally further comprises at least one protein.

Preferably, the at least one protein is a hydrophilic protein.

Preferably, the protein is soluble in water.

Preferably at least 90 wt.-%, more preferably at least 95 wt.-%, most preferably at least 98 wt.-% of the protein comprised by solution A is dissolved in solution A.

Preferably, the concentration of the at least one protein in solution A is 0.1 mg/ml to 3 mg/ml, more preferably the concentration is of 0.25 mg/ml to 2 mg/ml, most preferably of 0.5 mg/ml to 1 mg/ml.

The protein may be any protein known to the person skilled in the art.

The protein may have a molecular weight from 1 to 200000 g/mol, preferably 100 to 150000 g/mol, more preferably 500 to 100000 g/mol.

Furthermore, the protein may be selected from bovine serum albumin (BSA), IgG,. Leuprolide acetat, Insulin, human growth hormon (hGH), Desmopressin, Desoxiribonuclease I and γ-Interferon (γ-INF).

If at least one protein is present in step a), the method may comprise a further step d), following step c). In the optional step d), free protein is separated from particles comprising maltodextrin and protein. Preferably, this separation is achieved by centrifugation of the solution obtained after combination of solution A and B. Preferably, the centrifugation in step d) is carried out at 4 °C for 10 min to 2 h, more preferably at 4 °C for 20 min to 1 h, most preferably for 30 min. Preferably, the centrifugation is carried out at 600 rcf to 32000 rcf, more preferably at 3000 to 26000 rcf, most preferably at 6000 to 13000 rcf.

In step b), a solution B is provided, which comprises acetone and at least one poloxamer, preferably acetone and one poloxamer.

In solution B, acetone is an anti-solvent for maltodextrin. In the context of the invention anti-solvent for maltodextrin means that maltodextrin has a lower solubility in acetone than in water and may be used to initiate precipitation of maltodextrin from aqeous solutions.

Preferably, acetone is the only anti-solvent in solution B. Preferably, solution B comprises no other solvents or anti-solvents than acetone.

Preferably, solution B is obtained by solving at least one poloxamer in acetone. More preferably, solution B is obtained by solving one poloxamer in acetone.

A poloxamer is a non-ionic, tri-block copolymer surfactant, a poly(ethylene glycol)-*block-*poly(propylene glycol)-*block*-poly(ethylene glycol). The poloxamer may be selected for example from poloxamer 407, CAS(Chemical Abstracts Service) Registry Number: 9003-11-6; poloxamer 188, poloxamer 237, poloxamer 338, poloxamer 124, poloxamer 108, poloxamer 98, poloxamer 88, and poloxamer 68. Preferably the poloxamer is selected from poloxamer 407 and poloxamer 188. More preferably, the poloxamer is poloxamer 407 if solution A is added to solution B and the poloxamer is poloxamer 188 if solution B is added to solution A.

The poloxamer is used in an amount of 0.005 g /100 ml to 0.7 g/ 100 ml, preferably in an amount of 0.02 g /100 ml to 0.4 g/100 ml, more preferably in an amount of 0.025 g/ 100 ml.

In step c), solution A is combined with solution B.

Combining solution A with solution B may be performed by adding solution A to solution B or by adding solution B to solution A. Preferably, solution A is added to solution B.

Preferably, solution A is combined with solution B in a ratio of solution A: solution B of 1:50 to 1:4, preferably 1:30 to 1:3, more preferably 1:9 (v/v).

Preferably the solution A is combined with solution B at 0 to 30 °C, more preferably at 10 to 25 °C, most preferably at 20 to 25 °C.

Solution A may be combined with solution B in one portion, stepwise, or continuously. Preferably, combining solution A with solution B is performed stepwise or continuously. More preferably continuously.

Combining of solution A with solution B may be carried out by any suitable technical means known to the person skilled in the art. For example a syringe pump may be used.

Preferably combining solution A with solution B is carried out with an injection flow rate which is ranged from 10 ml/min to 50 ml/min, preferably from 25 ml/min to 40 ml/min, more preferably 35 ml/min while stirring.

In step c), if solution A comprises no protein, particles comprising maltodextrin and no protein are obtained by precepitation.

In step c) if solution A comprises at least one protein, particles comprising maltodextrin and at least one protein are obtained by precipitation. Preferably, the "particles comprising maltodextrin and at least one protein" comprise a protein which is encapsulated with "particles comprising maltodextrin and no protein".

If solution A comprises at least one protein, preferably at least 10 to 95 wt.-%, more preferably 30 to 80 wt.-%, most preferably 40 to 75 wt.-% and particularly preferred 70 wt.-% of the at least one protein of solution A is comprised by the particles comprising maltodextrin and at least one protein.

Further, the present invention is related to particles comprising maltodextrin and no protein, obtainable by the method as described above, wherein the solution A in step a) comprises no protein.

Preferably, the intensity-weighted mean diameter (z-average) particle size of the particle comprising maltodextrin and no protein is ranged from 50 nm to 200 nm, more preferably from 70 nm to 150 nm, most preferably from 80 nm to 100 nm. Wherein the intensity-weighted mean diameter (z-average) particle size is measured by dynamic light scattering.

The particles comprising maltodextrin and no protein may be used in a method for encapsulating other molecules, preferably proteins. The encapsulation may be performed by applying the method as decribed above or by application of methods known in the art. The encapsulated other molecules or proteins may be used in a drug delivery system.

Furthermore, the present invention is related to a particle comprising maltodextrin and at least one protein, obtainable by the method as desribed above, wherein the solution in step a) comprises the at least one protein.

Preferably, the intensity-weighted mean diameter (z-average) particle size of the particle comprising maltodextrin and at least one protein is ranged from 50 nm to 1000 nm, preferably from 100 nm to 500 nm, more preferably from 170 nm to 450 nm. Wherein the intensity-weighted mean diameter (z-average) particle size is measured by dynamic light scattering.

Preferably, in the particle comprising maltodextrin and least one protein, the loading rate, meaning the ratio of mass of protein to mass of maltrodextrin is ranged from 1 to 30 %, more preferably from 1 to 25%, most preferably from 1 to 21%, particular preferred from 1 to 11%.

The particles comprising maltodextrin and no protein may be used in a drug delivery system.

The drug deliver system may comprise pharmaceutically acceptable carriers. As used herein, "pharmaceutically acceptable carrier" includes any and all solvents, dispersion media, coatings, antibacterial and antifungal agents, isotonic and adsorption delaying agents, and the like that are physiologically compatible. The "pharmaceutically acceptable carrier" may be in the form of a solid, semisolid, liquid, or combinations thereof. Preferably, the carrier is suitable for enteral (such as oral) or parenteral administration (such as intravenous, intramuscular, subcutaneous, spinal or epidermal administration (e.g. by injection or infusion)).

Preferably, the polydispersity index of the particles comprising no protein and the particles comprising at least one protein is ranged from 0.01 to 0.3, preferably from 0.02 to 0.1, more preferably from 0.03 to 0.06.

A better understanding of the present invention and of its advantages will be had from the following examples, offered for illustrative purposes only. The examples are not intended to limit the scope of the present invention in any way.

### EXAMPLES OF THE INVENTION

### Materials

Three maltodextrins with different dextrose equivalents (DE 4.0 - 7.0, DE 13.0 - 17.0, and DE 16.5 - 19.5), α-lactose monohydrate (lactose), and bovine serum albumin (BSA) were obtained from Sigma Aldrich (St. Louis, USA). Poloxamer 407 was purchased from Caesar & Loretz GmbH (Hilden, Germany). Organic solvents of analytical grade (methanol, acetone) were bought from VWR (Bruchsal, Germany). A549 cells (ACC 107) were received from the DSMZ (Braunschweig, Germany) and cultured in Gibco RPMI 1640 from ThermoFisher Scientific (Waltham, USA). The medium was supplemented with 10% fetal calve serum (FCS) and 1% penicillin streptomycin from PAN (Aidenbach, Germany). Gibco Hanks' Balanced Salt Solution (HBSS) was bought from ThermoFisher Scientific. 3-(4,5-dimethylthiazol-2-yl)-2,5-diphenyltetrazolium bromide (MTT) was purchased from Roche (Mannheim, Germany).

### Example 1: Preparation of particles

Particles were prepared from maltodextrin *by* an inverse precipitation in methanol and acetone as anti-solvents. Briefly, maltodextrin was dissolved in milliQ water to obtain solutions of 5 mg/mL. A volume of 1 ml of the maltodextrin solution was added to 9 ml anti-solvent using a syringe pump (Legato, Kd scientific, U.S.A.), whilst stirring. The injection flow rate was set to 35 mL/min. Acetone has been used as anti-solvent, adding poloxamer 407 as stabilizer in various concentrations (0.1%, 0.05%, 0.025%, 0.01%). Samples were stirred for 15 min for equilibration before analysis.

### Physiochemical characterization of maltodextrin particles

The particles were analyzed with a focus on size, and size distribution, represented by the polydispersity index (Pdl), using the Zetasizer Nano ZS (Malvern, U.K.). Physicochemical properties were studied for 14 days to evaluate particle storage stability at room temperature. Particle morphology was examined by scanning electron microscopy (SEM EVO HD15, Carl Zeiss Microscopy GmbH, Jena, Germany). Therefore, samples were placed on a silica wafer, air-dried, and sputtered with a gold layer of approximately 15 nm thickness prior to imaging (Sputter coater: Quorum Q150R ES, Quorum Technologies Ltd, East Grinstead, UK). The accelerating voltage was 5 kV.

**Table 1: Preparation of maltodextrin particles, varying maltodextrin type, and stabilizer amount**

| | **Poloxamer [%]** | **Maltodextrin [mg/mL]** | **Poloxamer [mg]** | **Size [d.nm]** | **Pdl** |
|---|---|---|---|---|---|
| **Maltodextrin 4.0 - 7.0** | | | | | |
| A1 | 0.1 | 5 | 9 | 228.8 ± 7.6 | 0.157 |
| A2 | 0.05 | 5 | 4.5 | Aggregates | 0.555 |
| A3 | 0.025 | 5 | 2.25 | Aggregates | 0.792 |
| A4 | 0.01 | 5 | 0.9 | Aggregates | 0.847 |
| A5 | 0 | 5 | 0 | Aggregates | 0.926 |

| **Maltodextrin 13.0 - 17.0** | | | | | |
|---|---|---|---|---|---|
| B1 | 0.1 | 5 | 9 | 172.8 ± 17.8 | 0.049 |
| B2 | 0.05 | 5 | 4.5 | 174.8 ± 15.8 | 0.034 |
| B3 | 0.025 | 5 | 2.25 | 182 ± 15.6 | 0.035 |
| B4 | 0.01 | 5 | 0.9 | 221.7 ± 16.5 | 0.024 |
| B5 | 0 | 5 | 0 | 531.8 ± 149.3 | 0.077 |

| **Maltodextrin 16.5 - 19.5** | | | | | |
|---|---|---|---|---|---|
| C1 | 0.1 | 5 | 9 | 171.8 ± 4.5 | 0.055 |
| C2 | 0.05 | 5 | 4.5 | 210.9 ± 22.9 | 0.043 |
| C3 | 0.025 | 5 | 2.25 | 272 ± 70.7 | 0.028 |
| C4 | 0.01 | 5 | 0.9 | 446.8 ± 206.5 | 0.065 |
| C5 | 0 | 5 | 0 | 699.2 ± 348.9 | 0.124 |

As shown in table 1, Maltodextrin particles were prepared using different amounts of stabilizer (Poloxamer 407). Size and polydispersity index (PdI) were determined with the Zetasizer Nano ZS. The formation of particles using maltodextrin 4.0-7.0 was possible in presence of 9 mg stabilizer. Reducing the poloxamer amount resulted in aggregation of the particles (large size with high standard deviation and high PdI). Maltodextrins with an increased dextrose equivalent (>13) allowed a reproducible production of small particles. The size increased when reducing the poloxamer amount. The same procedure led to smaller particles for maltodextrin 13.0-17.0. Data is presented as mean ± SD of 3 batches.

Morphology was investigated using scanning electron microscopy (SEM). Images of maltodextrin DE 13.0 - 17.0 and DE 16.5 - 19.5 are shown in figure 2. Particles were spherical with a smooth surface and represented the size, determined by dynamic light scattering, using the Zetasizer Nano Z (Table 1). In contrast, SEM images of maltodextrin DE 4.0 - 7.0, especially with lower concentrations of poloxamer (< 0.1%) were characterized by heterogeneous areas which did not allow a serious interpretation (data not shown). However, particles could be detected for maltodextrin DE 4.0 - 7.0, stabilized with 0.1% poloxamer (Figure 5).

A reduction of stabilizer improved the visualization by SEM as no stabilizer covered the sample. Particles were shown in more detail and were homogenous in size and shape. The relatively small concentration of poloxamer 0.025% (B3 and C3) gave the impression of 'sticky' or 'melting' particles with some of them tending to fuse. This might be an artifact due to the drying process which is necessary for SEM sample preparation. However, it underlined the increasing destabilization of the particles as a consequence of poloxamer reduction.

### Example 2: Influence of the solvent and the stabilizer

**Table 2: Comparative experiments, using different solvents have been carried out, adding solution A to solution B at an injection rate of 35mL /min. Solution B has been stirred at 500 rds. P407 is polaxamer 407, and T85 is Tween 85.**

| **Concentration of maltodextrin in water** | **Ratio of volumes solution solution B the of A:** | **Stabilizer and Concentration of stabilizer [%]** | **Size and standard deviation [nm]** | **Polydispersity index (PdI)** |
|---|---|---|---|---|
| **Methanol** | | | | |
| 4mg/mL | 1:20 | | 14000±3613 | 1 |
| 4mg/mL | 1:20 | P407, 0.5% | 8213±923.5 | 1 |
| 4mg/mL | 1:20 | T85, 0.5% | 2379±586.5 | 0.874 |
| 4mg/mL | 1:5 | 0 | 4306±1587 | 1 |

| **Aceton** | | | | |
|---|---|---|---|---|
| 4mg/mL | 1:20 | 0 | 6836±2070 | 0.99 |
| 4mg/mL | 1:20 | P407, 0.5% | 118.3±1.153 | 0.106 |
| 4mg/mL | 1:5 | P407, 0.5% | 213.3±2.237 | 0.066 |
| 4 mg/mL | 1:5 | T85, 0.5% | 325.7±2 | 0.16 |

| **Ethyl actetate** | | | | |
|---|---|---|---|---|
| 4 mg/mL | 1:20 | 0 | Emulsion | - |
| 4 mg/mL | 1:5 | 0 | Emulsion | - |

As displayed in table 2, comparative experiments have been carried out in order to study the influence of the solvent, using different solvents, stabilizers, stabilizer concentrations and ratios of solution B to solution A. In case methanol is used as solvent, under all conditions only large particle sizes and corressponding PdI values of 0.874 and 1 could be obtained. In case of ethyl acetate only emulsions formed and no precipitation took place. Only the combination of aceton as solvent with a stabilizer resulted in smaller particle sizes with respective Pdl values of ≤ 0.2, meaning smaller particle sizes wherein the particle size distribution is sufficiently narrow.

### Example 3: Stability Tests

Storage stability at room temperature of all formulations was tested for 14 days. As expected, MD 4.0 - 7.0 showed no stability with high fluctuations in particle sizes, probably arising from the sedimentation of larger aggregations. Formulations of maltodextrin (DE > 13) were constant in size and PdI for at least 14 days (figure 1, table 3 and 4). However, if no stabilizer was applied, the fluctuation in particle size increased.

**Table 3: Size of maltodextrin particles on day of preparation (day 0), day 7 and day 14. Data is presented as mean ± SD of 3 batches. Particles were stable in size over 14 days.**

| **Poloxamer [%]** | **Day 1 Size [d.nm]** | **Day 7 Size [d.nm]** | **Day 14 Size [d.nm]** |
|---|---|---|---|
| **Maltodextrin particles 13.0 - 17.0** | | | |
| 0.1% | 172.8 ± 17.8 | 183.5 ± 17.9 | 185.4 ± 17.5 |
| 0.05% | 174.8 ± 15.8 | 181.7 ± 12.9 | 182.9 ± 12.2 |
| 0.025% | 182.0 ± 15.6 | 204.9 ± 30.1 | 209.5 ± 29.6 |
| 0.01% | 221.7 ± 16.5 | 244.1 ± 34.0 | 248.4 ± 36.5 |
| 0% | 531.8 ± 149.3 | 501.1 ± 120.3 | 479.4 ± 100.4 |

| **Maltodextrin particles 16.5 - 19.5** | | | |
|---|---|---|---|
| 0.1% | 171.8 ± 4.5 | 186.5 ± 11.3 | 194.1 ± 12.6 |
| 0.05% | 210.9 ± 22.8 | 243.2 ± 44.9 | 253.5 ± 46.1 |
| 0.025% | 272.0± 70.7 | 290.3 ± 78.8 | 295.9 ± 76.5 |
| 0.01% | 601.1 ± 62.8 | 574.1 ± 41.5 | 532.1 ± 4.7 |
| 0% | 875.9 ± 53.7 | 574.8 ± 284.4 | 472.1 ± 19.2 |

**Table 4: Polydispersity index (Pdl) of maltodextrin particles on day of preparation (day 0), day 7 and day 14. Data is presented as mean ± SD of 3 batches. Particles were stable regarding Pdl over 14 days.**

| **Poloxamer [%]** | **Day 1 Pdl** | **Day 7 PdI** | **Day 14 PdI** |
|---|---|---|---|
| **Maltodextrin particles 13.0 - 17.0** | | | |
| 0.1% | 0.05 | 0.03 | 0.03 |
| 0.05% | 0.03 | 0.02 | 0.03 |
| 0.025% | 0.04 | 0.02 | 0.03 |
| 0.01% | 0.02 | 0.02 | 0.02 |
| 0% | 0.08 | 0.09 | 0.10 |

| **Maltodextrin particles 16.5 - 19.5** | | | |
|---|---|---|---|
| 0.1% | 0.06 | 0.02 | 0.02 |
| 0.05% | 0.04 | 0.01 | 0.02 |
| 0.025% | 0.03 | 0.01 | 0.02 |
| 0.01% | 0.07 | 0.04 | 0.08 |
| 0% | 0.26 | 0.23 | 0.23 |

### Example 4: Cytotoxicity

Cell viability was evaluated using the 3-(4,5-dimethylthiazol-2-yl)-2,5-diphenyltetrazolium bromide (MTT) assay. For assessing the cytotoxicity of maltodextrin particles, 2x10⁴ A549 were seeded into 96 well plates. The cells were incubated with maltodextrin particles for 4 h and 24 h. Therefore, maltodextrin particles formulations (from maltodextrin DE 13.0 - 17.0, and DE 16.5 - 19.5, stabilized with 0.025% poloxamer) were dried after preparation by evaporation and dissolved in medium to obtain solutions of 5, 0.5, 0.05 and 0.005 mg/mL. As control, maltodextrin with a DE of 13.0 - 17.0 and 16.5 - 19.5, poloxamer 407 and α-lactose monohydrate, each dissolved in medium in the same concentrations, were also applied.

In all cases, following incubation of cells with particles or materials, cells were washed once with 200 µL HBSS buffer. A volume of 100 µL fresh HBSS buffer, containing 10% (v/v) MTT reagent (stock solution: 5 mg/mL) was added and incubated for 4 h. HBSS buffer was aspirated and 100 µL DMSO was added and incubated for 20 min. Absorbance was measured at 550 nm with the Tecan Infinite® 200 microplate reader (Tecan Deutschland GmbH, Germany). All incubation steps were performed at 37 °C. Cell viabilities were calculated in comparison to negative control (untreated cells as 100% value of cell viability) and positive control (1% Triton™ X-100 solution as 0% value of cell viability).

Lactose as well as poloxamer 407 showed cell viabilities around 100% for all tested concentrations. Maltodextrin particles did not show any cytotoxicity in the tested concentration range (table 5).

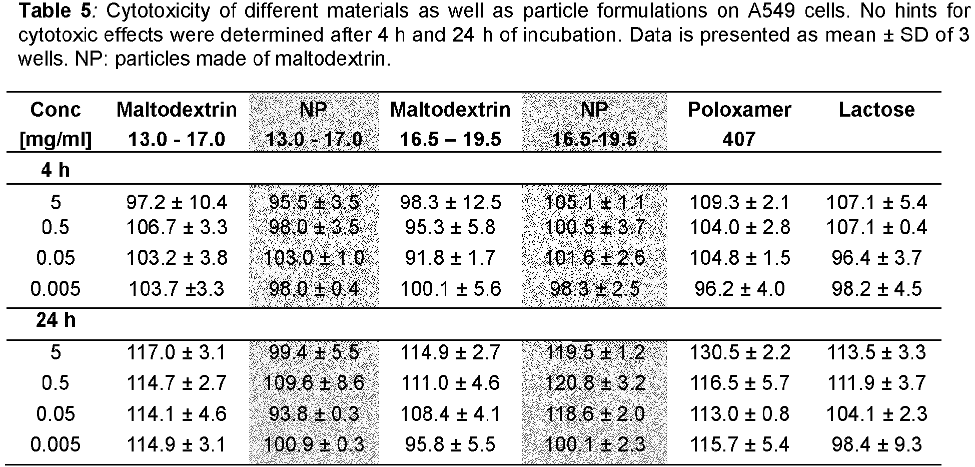

### Example 5: Loading of maltodextrin particles

Maltodextrin particles were loaded with BSA as model drug. The protein was added to the aqueous maltodextrin phase before particle preparation. BSA was dissolved in 5 mg/mL maltodextrin solution to obtain concentrations of 0.5 mg/mL and 1 mg/mL. Particles were prepared afterwards by adding 1 mL of the aqueous solution, containing the protein, to 9 mL acetone, containing 0.025% poloxamer 407 as stabilizer, using a syringe pump with an injection flow rate of 35 mL/min. For determining the protein encapsulation efficiency (EE) and loading rate (LR), loaded particles were separated from free protein using centrifugation at 9.500 rcf for 30 min at 4 °C (Rotina 420R, Hettich). The resulting pellet was dissolved in milliQ water and analysed via UV absorbance at 280 nm (BSA).

The encapsulation of proteins into maltodextrin particles was performed for the formulations B3 (MD 13.0 - 17.0) and C3 (MD 16.5 - 19.5), each containing 0.025% poloxamer as stabilizer. Encapsulation was performed as coprecipitation of maltodextrin and protein. Therefore, the protein was dissolved in maltodextrin solution, before inverse precipitation. Figure 3 emphasizes that particle size dramatically decreased from approx. 180 nm and 270 nm for blank maltodextrin 13.0 - 17.0 and 16.5 - 19.5, respectively, to approx. 80 nm, when BSA was encapsulated. The size of the loaded particles was independent from the maltodextrin type and slightly smaller for the higher amount of BSA. All formulations showed narrow size distributions, represented by a Pdl < 0.05. As control, BSA was precipitated without maltodextrin and formulations showed high Pdl values, indicating an inhomogeneous system.

Maltodextrin particles comprised approx. 70% of the applied BSA amount. No pellet was found after centrifugation of BSA controls precipitated without maltodextrin, emphasizing the successful encapsulation of BSA intro maltodextrin particles. A loading rate of 10% and 20% was quantified for the BSA in maltodextrin particles (0.5 g/L and 1 g/L, respectively).

### Example 6: Preparation of maltodextrin particles by addition of solution B to solution A

5 mg maltodextrin L (dextrose equivalent 16.5 - 19.5) were discolved in 1 ml Milli-Q-water. Further, 4.5 mg pluronic F-68 (poloxamer 188) was dissolved in 9 ml acetone (analytical grade). The pluronic solution was taken up in a 10 ml syringe (canula: 0.55 x 25 mm). 1 ml maltrodextrin solution was transferred in a snap-cap vial with stir bar (3x13 mm).

The 9 ml pluronic-acetone-solution was injected into the 1 ml maltodextrin-water-solution with the following settings:

| | |
|---|---|
| Diameter of the syringe: 16.11 mm | Injection rate: 35 mL/min |
| Volume: 9 ml | Stirring rate: 500 rpm |

50 µL sample was diluted with 950 µL acetone. The samples were measured with the Zetasizer in a glass cuvette (Maltodextrin size SOP). Three samples were prepared according the procedure as decribed above.

**Table 6: Size and polydispersity index (PDI) of the three samples prepared. The values represent the average value of three measurements carried for each of the three samples.**

| **Batch** | **Size [nm]** | **PDI** |
|---|---|---|
| 1 | 322.7 | 0.164 |
| 2 | 327.6 | 0.138 |
| 3 | 410.5 | 0.169 |

### REFERENCES

(1) Devineni D et al.; Journal of microencapsulation, 2007;24(4):358-70. PubMed PMID: 17497389.
(2) Jallouli Y et al.; International journal of pharmaceutics, 2007, 344(1-2):103-9. PubMed PMID: 17651930.
(3) H. Fessi, F. Puisieux, J.P. Devissaguet, N. Ammoury, S. Benita, Nanocapsule formation by interfacial polymer deposition following solvent displacement, International Journal of Pharmaceutics, 55 (1989) R1-R4.
(4) Tan Y et al.; ACS applied materials & interfaces, 2009,1(4):956-9. PubMed PMID: 20356023.
(5) Qiu C. et al.; Food Science and Technology, 2017, 164-171
(6) Dimier-Poisson I. et al.; Biomaterials, 2015, (50), 164-175
(7) Paillard A. et al.; Pharmaceutical Research, 2010, (1), 126-133
(8) Bernocchi B. et al.; Journal of Controlled Release, 2016, (232), 42-50

## Claims

1. A method for the preparation of particles, comprising the steps:
a) providing a solution A, which comprises water and maltodextrin of the types DE 13.0 -17.0 or DE 16.5 -19.5;
b) providing a solution B, which comprises acetone and at least one poloxamer ;
c) combining solution A with solution B.

2. The method for preparation of particles of claim 1, wherein the solution A in step a) further comprises at least one protein.

3. The method of claim 2, wherein the concentration of the at least one protein in solution A is 0.1 mg/ml to 3 mg/ml, preferably of 0.25 mg/ml to 2 mg/ml, more preferably of 0.5 mg/ml to 1 mg/ml.

4. The method of any one of claims 2 to 3, wherein the at least one protein is a hydrophilic protein.

5. The method of any one of claims 1 to 4, wherein
I) solution A comprises maltodextrin solved in water, in a concentration ranged from 0.5 mg/mL to 15 mg/ml, preferably from 1 mg/ml to 10 mg/ml, more preferably in a concentration of 5 mg/ml and/or
II) combining in step c) is carried out by adding solution A to solution B and/or
III) the ploxamer is selected from poloxamer 407, poloxamer 188, poloxamer 237, poloxamer 338, poloxamer 124, poloxamer 108, poloxamer 98, poloxamer 88, and poloxamer 68, preferably from poloxamer 407 and poloxamer 188.

6. The method of any one of claims 1 to 5, wherein poloxamer 407 is used in an amount of 0.005 g /100 ml to 0.7 g/ 100 ml, preferably in an amount of 0.02 g /100 ml to 0.4 g/100 ml, more preferably in an amount of 0.025 g/ 100 ml.

7. The method of any one of claims 1 to 6, wherein the ratio of solution A to solution B is 1:50 to 1:4, preferably 1:30 to 1: 3, more preferably 1:9 (v/v).

8. The method of any of claims 1 to 7, wherein the injection flow rate is ranged from 10 ml/min to 50 ml/min, preferably from 25 ml/min to 40 ml/min, more preferably 35 ml/min while stirring.

9. The method of any one of claims 1 to 8, wherein the polydispersity index of the particles is ranged from 0.01 to 0.3, preferably from 0.02 to 0.1, more preferably from 0.03 to 0.06.

10. Particles, comprising maltodextrin and no protein, obtainable by the method according to claim 1 and 5 to 9.

11. Particles, comprising maltodextrin and at least one protein, obtainable by the method according to claim 2 to 9.

12. Use of the particles of claim 10 in a method for encapsulating proteins.

13. The method of any one of claims 1, 5 to 9 and the particles of claim 10, wherein the particle comprises no protein and the intensity-weighted mean (z-average) particle size of the particles is ranged from 50 nm to 200 nm, preferably from 70 nm to 150 nm, more preferably from 80 nm to 100 nm.

14. The method of any one of claims 2 to 9 and the particles of claim 11, wherein the intensity-weighted mean (z-average) particle size of the particles is ranged from 50 nm to 1000 nm, preferably from 100 nm to 500 nm, more preferably from 170 nm to 450 nm.

15. Use of the particles of claim 11 in a drug delivery system.
